# EUROPEAN PATENT APPLICATION

(11) **EP 4 390 957 A1**
(43) Date of publication of application: **26.06.2024**
(21) Application number: 23220157.4
(22) Date of filing: 22.12.2023
(51) Int. Cl.: G16H 20/40, A61B 90/90, G16H 30/40, G16H 40/63

(54) **SYSTEMS AND METHODS FOR AUTOMATICALLY CONFIGURING SURGICAL TOOLS AND SURGICAL INPUT DEVICES**

(30) Priority: 22.12.2022 US 202263476940 P
(71) Applicant: Stryker Corporation, Kalamazoo, MI 49002 (US)
(72) Inventor: WOOLFORD, Brady Lewis, Kalamazoo, 49002 (US); ABDULQADER, Dana Emad Mohamed, Kalamazoo, 49002 (US); DAHLBY, Ryan David, Kalamazoo, 49002 (US)
(74) Representative: V.O.

(57) **Abstract**

Systems and methods for automatically configuring a surgical tool and a surgical input device for the surgical tool are provided. A system receives one or more images and identifies, based at least in part on the one or more images, the surgical tool from a plurality of surgical tools. Identifying the surgical tool from the plurality of surgical tools may comprise identifying the surgical tool from the plurality of surgical tools based at least in part on classification output data generated by one or more classifiers applied to the one or more images. The system generates an instruction to pair the surgical input device with the surgical tool.

## Description

### CROSS-REFERENCE TO RELATED APPLICATIONS

This application claims the benefit of U.S. Provisional Application No. 63/476,940, filed December 22, 2022, the entire contents of which are hereby incorporated by reference herein.

### FIELD

The present application relates generally to surgical tools and surgical input devices, and more specifically to systems and methods for automatically pairing and configuring surgical tools and surgical input devices based on intraoperative images.

### BACKGROUND

An operating room is typically crowded with multiple input devices for controlling surgical equipment (referred to herein as surgical input devices), including, for example, foot switches and hand controls that can control different tools that a surgeon might use during a surgical procedure. Sometimes, a surgical input device may be used to control different tools during a surgical procedure. However, such arrangements typically require users to manually pair/unpair the surgical input device to/from the different tools, which may be time-consuming, imprecise, and prone to human error.

### SUMMARY

Disclosed herein are systems and methods for automatically pairing a surgical input device to an identified surgical tool, and for automatically configuring one or both of the surgical input device and the identified surgical tool, based on one or more images (e.g., endoscopic images, intraoperative video data, and the like) received by the system. The system may receive one or more images such as real-time video data of a surgical environment. Based on the one or more images, the system may automatically identify a surgical tool from a plurality of surgical tools, for example by processing the received one or more images using one or more classifiers and by identifying the surgical tool based on classification output data generated by one or more of the classifiers. The system may then automatically pair the identified surgical tool with the surgical input device, such that the surgical input device can be used to control the surgical tool.

Based on the one or more images, the system may further automatically select a functionality for the identified surgical tool, and may automatically set the identified surgical tool to the selected functionality. Based on the one or more images, the system may further automatically select a modality for the surgical input device, and may automatically set the surgical input device to the selected modality.

In this manner, one or more computerized algorithms and/or machine learning classifiers may be leveraged to enable a system to automatically pair a surgical tool to a surgical input device, set a surgical tool to a selected functionality, and set the input device to a selected modality. Any one or more of these functionalities may be performed on the basis of analysis of one or more images of a surgical environment, allowing for more reliable and rapid surgical procedures without requiring a user to manually manage surgical tool pairing, surgical tool functionality settings, and input device modality settings. Furthermore, use of the systems and methods described herein may greatly reduce the number of surgical input devices that are required to be present in an operating room, even allowing for use of a single universal surgical input device, thereby reducing clutter, confusion, and sources of potential user error.

In some aspects, a system for automatically configuring a surgical tool and a surgical input device for the surgical tool is provided, the system comprising one or more processors configured to cause the system to: receive one or more images; identify, based at least in part on the one or more images, the surgical tool from a plurality of surgical tools; and generate an instruction to pair the surgical input device with the surgical tool.

Optionally, the one or more processors are further configured to cause the system to transmit the instruction to pair the surgical input device with the surgical tool to one or both of the surgical input device and the surgical tool.

Optionally, the one or more processors are further configured to cause the system to, after identifying the surgical tool: receive a confirmatory user input confirming the identification of the surgical tool; and in response to receiving the confirmatory user input confirming the identification of the surgical tool, transmit the instruction to pair the surgical input device with the surgical tool to one or both of the surgical input device and the surgical tool.

Optionally, the confirmatory user input confirming the identification of the surgical tool is received from the surgical input device.

Optionally, the one or more processors are configured to apply one or more classifiers to the one or more images, each of the one or more classifiers generating classification output data; and identifying the surgical tool from the plurality of surgical tools based at least in part on the one or more images comprises identifying the surgical tool from the plurality of surgical tools based at least in part on the classification output data.

Optionally, the one or more classifiers comprise at least one of: a tool identification classifier; a tool location classifier; an anatomical identification classifier; an anatomical position classifier; a surgery type classifier; a surgery stage classifier, a tool identification classifier, and/or a tool location classifier.

Optionally: the one or more processors are configured to apply one or more computer vision algorithms to the one or more images, each of the one or more computer vision algorithms generating computer vision output data; and identifying the surgical tool from the plurality of surgical tools based at least in part on the one or more images comprises identifying the surgical tool from the plurality of surgical tools based at least in part on the computer vision output data.

Optionally, the one or more processors are further configured to cause the system to: select, based at least in part on the one or more images, a functionality for the surgical tool; and generate an instruction to configure the surgical tool in accordance with the functionality.

Optionally, the one or more processors are further configured to cause the system to transmit the instruction to configure the surgical tool in accordance with the functionality to the surgical tool.

Optionally, the one or more processors are further configured to cause the system to, after selecting the functionality for the surgical tool: receive a confirmatory user input confirming the selection of the functionality for the surgical tool; and in response to receiving the confirmatory user input confirming the selection of the functionality for the surgical tool, transmit the instruction to configure the surgical tool in accordance with the selected functionality to the surgical tool.

Optionally, the confirmatory user input is received from the surgical input device.

Optionally: the one or more processors are configured to cause the system to, before selecting the functionality for the surgical tool, receive data representing a user preference; and selecting the functionality for the surgical tool is based at least in part on the data representing the user preference.

Optionally: receiving the data representing the user preference comprises: accessing a user profile electronically storing one or more user preferences associated with a user; and identifying the user preference, from the one or more user preferences associated with the user, indicating a preferred functionality; and selecting the functionality for the surgical tool is based at least in part on the preferred functionality.

Optionally, the one or more processors are further configured to cause the system to: select, based at least in part on the one or more images, a modality for the surgical input device; and generate an instruction to configure the surgical input device in accordance with the modality.

Optionally, the modality is selected based at least in part on a selected functionality for the surgical tool.

Optionally, the one or more processors are further configured to cause the system to transmit the instruction to configure the surgical input device in accordance with the modality to the surgical input device.

Optionally, the one or more processors are further configured to cause the system to, after selecting the modality for the surgical input device: receive a confirmatory user input confirming the selection of the modality for the surgical input device; and in response to receiving the confirmatory user input confirming the selection of the modality for the surgical input device, transmit the instruction to configure the surgical input device in accordance with the modality to the surgical input device.

Optionally, the confirmatory user input confirming the selection of the modality for the surgical input device is received from the surgical input device.

Optionally: the one or more processors are configured to cause the system to, before selecting the modality for the surgical input device, receive data representing a user preference; and selecting the modality for the surgical input device is based at least in part on the data representing the user preference.

Optionally: receiving the data representing the user preference comprises: accessing a user profile electronically storing one or more user preferences associated with a user; and identifying the user preference, from the one or more user preferences associated with the user, indicating a preferred modality; and selecting the modality for the surgical input device is based at least in part on the preferred modality.

Optionally, the surgical input device comprises a foot switch.

Optionally, the surgical input device comprises a hand control device.

Optionally, the one or more images comprise intraoperative images captured by a surgical imaging device, the one or more images including the surgical tool within a field of view of the surgical imaging device.

In some aspects, a method for automatically configuring a surgical tool and a surgical input device for the surgical tool is provided, the method comprising: receiving one or more images; identifying, based at least in part on the one or more images, the surgical tool from a plurality of surgical tools; and generating an instruction to pair the surgical input device with the surgical tool.

In some aspects, a non-transitory computer-readable storage medium storing instructions for automatically configuring a surgical tool and a surgical input device for the surgical tool is provided, the instructions configured to be executed by one or more processors of a system to cause the system to: receive one or more images; identify, based at least in part on the one or more images, the surgical tool from a plurality of surgical tools; and generate an instruction to pair the surgical input device with the surgical tool.

In some aspects, a computer program product comprising software code portions for automatically configuring a surgical tool and a surgical input device for the surgical tool is provided, the software code portions configured to be executed by one or more processors of a system to cause the system to: receive one or more images; identify, based at least in part on the one or more images, the surgical tool from a plurality of surgical tools; and generate an instruction to pair the surgical input device with the surgical tool.

It will be appreciated that any of the variations, aspects, features and options described in view of the systems apply equally to the methods and vice versa. It will also be clear that any one or more of the above variations, aspects, features and options can be combined.

### BRIEF DESCRIPTION OF THE FIGURES

The invention will now be described, by way of example only, with reference to the accompanying drawings, in which:
FIG. 1 shows a system 100 for automatically configuring a surgical tool and an input device for the surgical tool, according to some aspects.
FIG. 2 shows a functional block diagram of an exemplary image processing system, according to some aspects.
FIG. 3 depicts a method 300 for automatically configuring a surgical tool and an input device for the surgical tool, according to some aspects.
FIG. 4 shows a computing system 400, according to some aspects.
FIG. 5A illustrates an example of the detection of the presence of a surgical tool in an endoscopic video frame, according to some aspects.
FIG. 5B illustrates an example of the detection of the presence of a feature associated with the clarity of an endoscopic video frame, according to some aspects.

### DETAILED DESCRIPTION

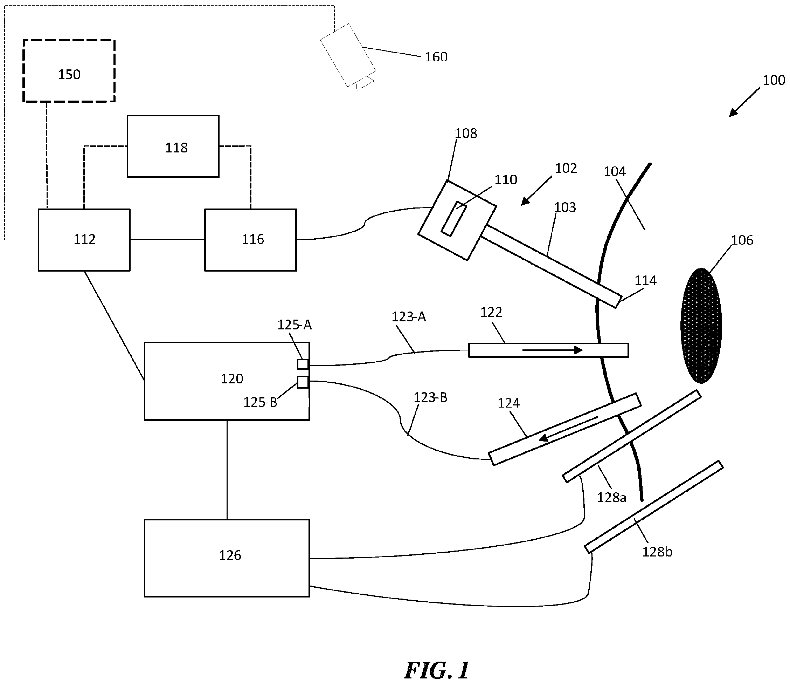

FIG. 1 illustrates an exemplary endoscopy system according to examples of the disclosure. System 100 includes an endoscopic imaging device 102 for insertion into a surgical cavity 104 for imaging tissue 106 within the surgical cavity 104 during a medical procedure. The endoscopic imaging device 102 may include an endoscope 103 that extends from an endoscopic camera head 108 that includes one or more imaging sensors 110. Light reflected and/or emitted (such as fluorescence light emitted by fluorescing targets that are excited by fluorescence excitation illumination light) from the tissue 106 is received by the distal end 114 of the endoscope 103. The light is propagated by the endoscope 103, such as via one or more optical components (for example, one or more lenses, prisms, light pipes, or other optical components), to the camera head 108, where it is directed onto the one or more imaging sensors 110. In one or more examples, one or more filters (not shown) may be included in the endoscope 103 and/or camera head 108 for filtering a portion of the light received from the tissue 106 (such as fluorescence excitation light). While the example above describes an example implementation of an endoscopic imaging device, the example should not be seen as limiting to the disclosure and the systems and methods described herein can be implemented using other imaging devices that are configured to image an internal area of a patient, an external area of a patient, and/or a surgical environment including the patient and/or the surrounding operating room (OR).

The one or more imaging sensors 110 generate pixel data that can be transmitted to a camera control unit 116 that is communicatively connected to the camera head 108. The camera control unit 116 generates a video feed from the pixel data that shows the tissue being viewed by the endoscopic imaging device 102 at any given moment in time. In one or more examples, the video feed can be transmitted to an image processing unit 112 for further image processing, storage, display, and/or routing to one or more remote computing systems 150 such as a cloud computing system. The video feed or portions thereof can be transmitted to one or more displays 118, from the camera control unit 116 and/or the image processing unit 112, for visualization by medical personnel, such as by a surgeon for visualizing the surgical cavity 104 during a surgical procedure on a patient.

The image processing unit 112 can be communicatively coupled to an endoscopic surgical pump 120 configured to control the inflow and outflow of fluid in an internal portion of a patient. As described in further detail below, the image processing unit 112 can use the video data it processes to determine an adjusted pressure setting for the surgical pump 120, usable for regulating the pressure at an internal area of a patient such as surgical cavity 104. The image processing unit 112 may receive and process video and/or image data generated by imaging devices other than the endoscopic imaging device 102. For example, the image processing unit 112 may receive video and/or image data generated by one or more cameras 160 that captures video and/or images from outside of the body of the patient. The video and/or images generated by the one or more cameras 160 may capture the use of one or more tools by a user performing a surgical procedure on the patient. The one or more cameras 160 can include, for example, a pan-tilt-zoom camera, an in-light camera, and/or a stereoscopic camera that generates three-dimensional imaging data. The image processing unit 112 can use the video data it processes to control the surgical pump 120 so as to regulate one or more characteristics (e.g., pressure and/or flow rate) of flow in an internal area of a patient such as surgical cavity 104. The surgical pump 120 can include an inflow instrument 122 configured to deliver a clear fluid such as saline into the surgical cavity 104 via a fluid supply line 123-A. The surgical pump 120 can also include a dedicated suction instrument 124 configured to suction fluid out of the surgical cavity 104 via a suction line 123-B. In one or more examples, the surgical pump 120 is configured to regulate the internal pressure of the surgical cavity by either increasing or decreasing the rate at which the inflow instrument 122 pumps fluid into the surgical cavity 104 and/or by increasing/decreasing the amount of suction at suction instrument 124. This can be done, for example, via an inflow control system 125-A (for example, a dedicated pump and/or valve system) for the inflow instrument 122 and/or dedicated suction control system 125-B (that includes, for example, a dedicated pump and/or valve system) for the suction instrument 124. In one or more examples, the surgical pump can also include a pressure sensor that is configured to sense the pressure inside of surgical cavity 104 during a surgical procedure.

In one or more examples, the system 100 can also include an input device 126 that is configured to control and/or operate one or both of a plurality of tools including tools 128a and 128b used in performing a minimally invasive surgical procedure in the surgical cavity 104. In one or more examples, the input device 126 (and/or the tools 128a-128b themselves) may be communicatively coupled to the surgical pump 120. By communicatively coupling the tools 128a-b and/or input device 126 the surgical pump 120, the surgical pump 120 can coordinate the actions of its own dedicated suction instrument 124 as well as functions of the tools 128a-b to regulate the pressure of the surgical cavity 104 as well as to coordinate and control other surgical operations performed by tools 128a-b. As used herein, the term surgical pump (e.g., surgical pump 120) encompasses a single device (e.g., a single off-the-shelf device) that provides the fluid inflow and suction and multiple communicatively interconnected devices that collectively provide controlled fluid inflow and suction. As used herein, the terms tool and surgical tool encompass inflow instruments that provide fluid to the surgical cavity, dedicated suction instruments, and other surgical tools such as, but not limited to cutters, burs, resection devices, RF probes, and other tools.

As described further herein, different conditions taking place inside of surgical cavity 104 and/or elsewhere in the surgical environment can call for adjusting the manner in which input device 126 is paired to (e.g., communicatively coupled with and configured to control) one or both of surgical tools 128a-b, the manner in which one or both of surgical tools 128a-b are set to one or more functionalities, and/or the manner in which one or both input device 126 is set to one or more modalities. For example, different conditions may call for pairing input device 126 from one or more of surgical tools 128a-b, unpairing input device 126 from one or more of surgical tools 128a-b, setting one or more of surgical tools 128a-b to a certain functionality (e.g., setting it to a certain mode), and/or setting input device 126 to a certain modality (e.g., mapping one or more buttons or pedals of input device 126 to different functionalities of one or more of surgical tools 128a-b in a certain manner).

Conventionally, a user would need to recognize that conditions require an update in settings of input device 126 and/or surgical tools 128a-b, and would then manually adjust said settings. This process can interrupt the surgical procedure itself as the user would need to stop with the procedure to make the necessary adjustments, and further requires that the user constantly assess whether the settings are correct for the given conditions of the surgery.

Automating the process of detecting features in one or more image frames that indicate conditions associated with updating settings, as well as the process of updating said settings, can thus reduce the cognitive load of the user, but in one or more examples can also ensure that the settings of input devices and surgical tools are controlled with precision.

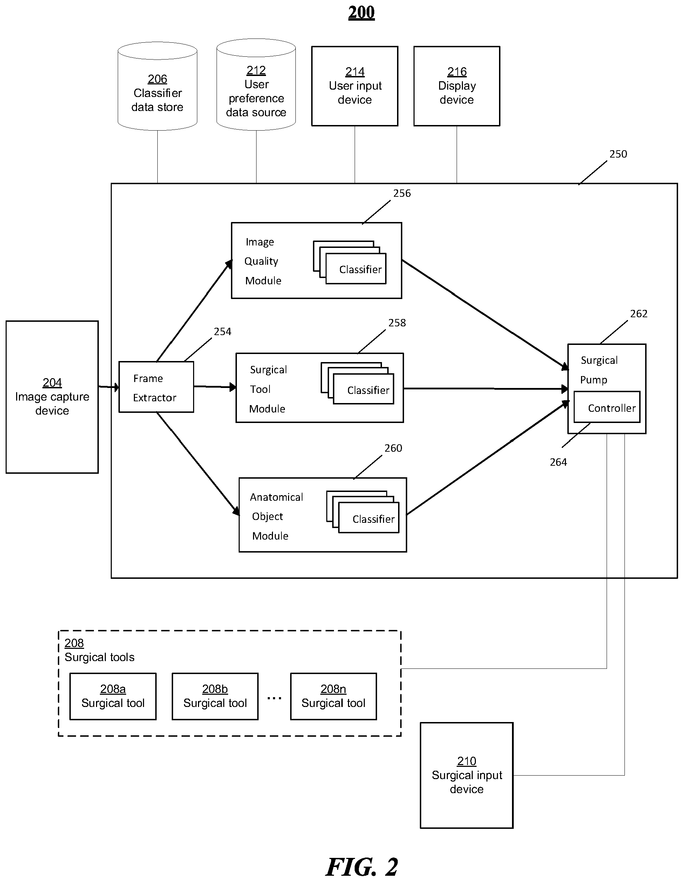

FIG. 2 shows a system 200 for automatically configuring a surgical tool and an input device for the surgical tool. In some aspects, all or part of system 200 may be provided as a part of, or in place of all or part of, system 100. In some aspects, any components and/or functionality of system 100 and 200 may be combined in whole or in part with one another.

As shown, system 200 includes image processing system 250 communicatively coupled (e.g., by one or more wired and/or wireless network communication protocols and/or by one or more bus lines) to a plurality of additional components of system 200, including image capture device 204, classifier(s) data store 206, surgical tools 208, surgical input device 210, user preference data source 212, user input device 214, and display device 216. Any one or more components of image processing system 250 may be provided as part of a single computing device, multiple computing devices, a distributed computing system, one or more servers, and/or one or more cloud computing systems. Image processing system 250 may be provided in a single housing along with any one or more of the other components of system 200, for example by being provided inside a housing of image capture device 204 or by being provided inside a housing of surgical input device 210.

Image processing system 250 may be configured to execute instructions stored on one or more non-transitory computer-readable storage mediums, wherein said instructions may cause system 200 to execute all or part of any one or more of the methods described herein.

Image processing system 250 may be communicatively coupled to surgical tools 208 and to surgical input device 210. Surgical tools 208 and surgical input device 210 may share any one or more characteristics in common with surgical tools 128a-b and input device 126 of FIG. 1, respectively. Communicative coupling may be by any wired and/or wireless communication protocol, for example including Stryker Firewire Bus (SFB), Ethernet, WiFi, Bluetooth, and/or USB. In some examples, image processing system 250 may be communicatively coupled to surgical tools 208 and to surgical input device 210 via surgical pump 262, as shown in the example of FIG. 2. As described herein, system 200 may execute instructions that cause system 200 to facilitate the configuration of one or more of surgical tools 208 and surgical input device 210 for use with one another.

Surgical tools 208 may include surgical tools 208a, 208b, and 208n. Surgical tools 208 may share any one or more characteristics in common with surgical tools 128a-128b of FIG. 1. Surgical tools 208 may include any suitable number of surgical tools. Surgical tools 208 may include one or more cutting tools, resection tools, drilling tools, probes, sinuscopes, cauterization tools, or the like. One of more of surgical tools 208 may be a handheld tool that is held during use by a user (e.g., surgeon, medical staff, and the like).

Surgical input device 210 may be an input device that is physically separate from surgical tools 208 and configured to communicate (directly and/or indirectly) with surgical tools 208 via wired and/or wireless communication. Surgical input device 210 may share any one or more characteristics in common with input device 126 of FIG. 1. Surgical input device 210 may include a handheld input device having one or more actuatable buttons, touch-sensitive buttons, knobs, dials, keys, triggers, accelerometers, gyroscopes, or the like. Surgical input device 210 may include a foot-operable device (e.g., a foot switch) including one or more pedals configured to be depressed by a foot of a user. Surgical input device 210 may include both hand controls and foot controls. Surgical input device 210 may include one or more buttons configured to toggle surgical input device 210 between a plurality of different modes.

Image capture device 204 may include any image sensor (e.g., camera) configured to capture one or more images (e.g., video data) of the surgical environment including one or more of surgical tool(s) 208. Image capture device 204 may share any one or more characteristics in common with endoscopic imaging device 102 and/or camera 160 of FIG. 1. Image capture device 204 may include an arthroscopic device and/or an endoscopic device. As used herein, a surgical environment may refer to an internal area of a patient (e.g., in the case of endoscopic use cases) and/or to an area surrounding a patient (e.g., in the case of non-endoscopic use cases and/or endoscopic use cases in which images of a scene external of a patient are captured, such as using the camera 160), for example including any area within an OR. The surgical environment may optionally include one or more additional components of system 200, including for example surgical input device 210. Image capture device 204 may capture one or more images (e.g., video data) and may transmit the captured one or more images to image processing system 250 for processing as described herein. The captured one or more images may be transferred in real time (e.g., on a frame-by-frame basis as video is captured).

Classifier data store 206 may include any computer-readable storage medium storing one or more classifiers (e.g., machine learning classifiers). The classifiers may be retrieved for use by image processing system 250 in analyzing captured sets of one or more images received by image processing system 250 from image capture device 204. The one or more classifiers stored on classifier data store 206 may be configured to allow image processing system 250 to receive one or more images as input, apply the one or more classifiers to the received one or more images, and generate classification output data that indicates one or more numerical scores or inference values for respective classifications based on the received one or more images.

The one or more classifiers may include a tool identification classifier configured to generate classification output data identifying a surgical tool, a tool location classifier configured to generate classification output data identifying a location of a surgical tool, an anatomical identification classifier configured to generate classification output data identifying an anatomical feature, an anatomical position classifier configured to generate classification output data identifying a position of an anatomical structure, a surgery-type classifier configured to generate classification output data identifying a surgery type, a surgery-stage classifier configured to generate classification output data identifying a surgery stage, an implant classifier configured to generate classification output data identifying an implant, and/or an implant location classifier configured to generate classification output data identifying a location of an implant. Additionally or alternatively, an object detection algorithm may be used to generate an output specifying both an object type and a location of the object with respect to an internal portion of a patient. The output generated by an object detection algorithm may be used in addition to or alternatively to the classification output data described above or described elsewhere herein, and may be used in any manner in which classification output data may be used as described herein. In some examples, object detection algorithms may be provided by Region-based Convolutional Neural Network (R-CNN) architectures and/or by You Only Look Once (YOLO) network architectures.

As described in further detail herein, classification output data generated using one or more classifiers stored on classifier data store 206 may be used by image processing system 250 to identify a surgical tool from surgical tools 208, to select a functionality for an identified surgical tool, and/or to select a modality for an input device configured to be used with the identified surgical tool.

As used herein, a functionality for a surgical tool may refer to a mode and/or manner of operation for the surgical tool. A surgical tool may be toggled between different functionalities, such that it may be set to one functionality (or to a set of functionalities) at a time, and/or such that one or more functionalities may be selectively activated and/or deactivated. For example, a resection device may be configured to operate in accordance with a plurality of different functionalities including a forward drilling functionality, a high RPM functionality, an oscillation functionality, and/or a low-RPM functionality. In another example, an RF probe may be configured to operate in accordance with plurality of different functionalities including a resection functionality, a coagulation functionality, and/or different functionalities corresponding to different power levels. In another example, a sinuscope may be configured to operate in accordance with a plurality of different functionalities, including a rinse functionality, a pulse functionality, and/or a suction functionality. In another example, a surgical tool may be configured to operate in accordance with a plurality of different functionalities including a drilling functionality, a bone cutting functionality, and/or an implant screwing functionality. In another example, a surgical drill may be configured to operate in accordance with a plurality of different functionalities including a forward drilling functionality (in which a drill bit of the surgical drill rotates clockwise) and a backward drilling functionality (in which the drill bit of the surgical drill rotates counterclockwise).

As used herein, a modality for an input device may refer to the manner in which the input device is mapped to a surgical tool. For example, different buttons or switches of a surgical input device may be mapped to different functionalities of a surgical tool. In one example, a first pedal of a surgical input device (e.g., a foot switch) may be mapped to a forward drilling functionality, while a second pedal of the surgical input device may be mapped to a backward drilling functionality. In another example, a first modality of a surgical input device may map a given pedal to a start/stop functionality of a pump, while a second modality of the surgical input device may map the given pedal to a wash functionality of the pump.

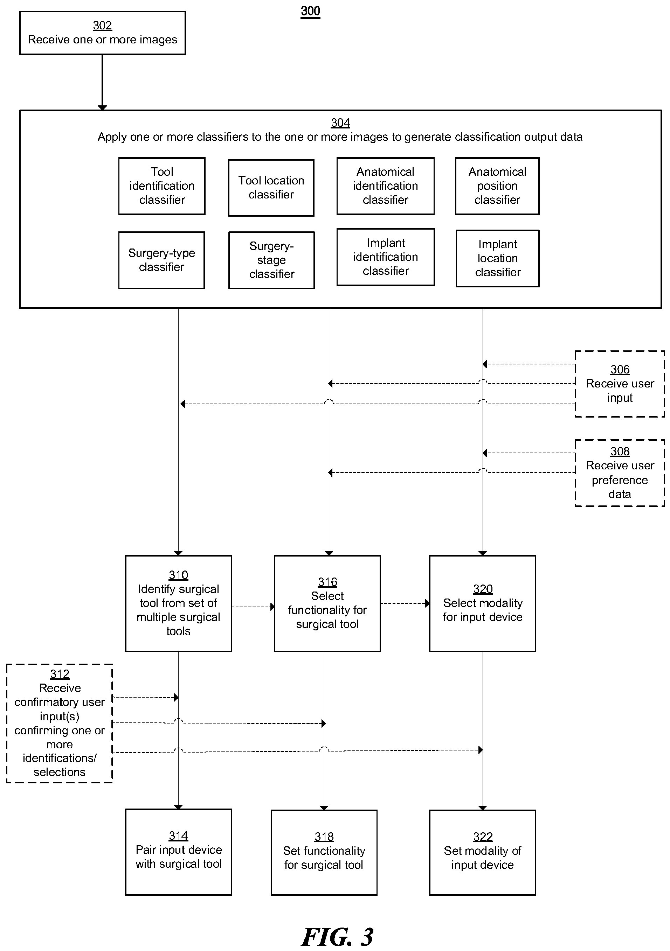

Image processing system 250 can include one or more processors (e.g., one or more of image processing system 250) and may perform one or more steps of process 300 of FIG. 3, such as steps 302 and/or 304 as described below. As discussed further below, image processing system 250 may extract image frames from video (e.g., as captured by image capture device 204) and analyze the image frames using one or more machine learning models to determine the presence of features in the image frames that correspond with conditions in a surgical environment in response to which adjustments to pairings between input devices and surgical tools, adjustments to functionality settings for surgical tools, and/or adjustments to modality settings for input devices may be desired. The image processing system 250 can include a single processor or multiple processors. The image processing system 250 can be provided as a single computing system, such as image processing unit 112 of FIG. 1, or can include multiple communicatively coupled computing systems, such as a combination of image processing unit 112 and remote computing system 150. Different functions of image processing system 250 can be implemented by different computing systems that are communicatively coupled to one another. For example, image frame extraction from video can be implemented by a local computing system that is located in an operating room and one or more machine learning models can be implemented by one or more remote computing systems, such as a cloud computing system, that receive the image frames from the local computing system and return information regarding detected features to the local computing system. The one or more processors of image processing system 250 can include one or more CPUs, one or more GPUs, one or more TPUs, and/or one or more FPGAs.

In one or more examples, the image processing system 250 receives video data from image capture device 204. In one or more examples, the image processing system 250 implements a frame extractor 254 for extracting one or more image frames from the received video data. Additionally, or alternatively, image processing system 250 may receive one or more images directly (e.g., from camera control unit 116, image processing unit 112, remote computing system 150, and the like), obviating the need for utilizing frame extractor 254. The image processing system 250 may implement one or more machine learning models for determining what features (if any) are present in a given image frame or set of image frames that may be associated with one or more conditions requiring (e.g., as indicated by one or more stored rule sets) adjustment to the pairings, functionality settings, and/or modality settings. The image processing system 250 may implement multiple modules, each having its own machine learning model(s), or classifier(s), for determining the presence of different types of features. In the illustrated example, the image processing system 250 may include an image quality module 256 that may implement one or more classifiers for determining whether features associated with poor image quality are present in the image frames, a surgical tool module 258 that may implement one or more classifiers for determining whether one or more surgical tools are present in the image frames, and/or an anatomical object module 260 that may implement one or more classifiers for determining whether one or more anatomical objects are present in the image frames. The one or more classifiers of each module can each generate classification output data indicating the presence or absence of features in the one or more image frames. In one or more examples, each module may make determinations regarding the presence or absence of features in the one or more images frames by comparing the classification output data generated by the one or more classifiers to predetermined thresholds. For example, a module may determine that a given feature is present in one or more image frames when classification output data generated by a classifier is above a predetermined threshold. The classification output data generated by the one or more classifiers may comprise one or more inference values. In one or more examples, classification output data is provided as output to an external system for the external system to make determinations regarding the presence or absence of features in the one or more images frames by comparing the classification output data generated by the one or more classifiers to predetermined thresholds. As used herein, the term "module" refers to a software module (e.g., a machine learning model) and is not intended to refer to particular hardware. It will be understood by a person of ordinary skill that different modules can be implemented on the same computing hardware, on different computing hardware, or a combination thereof.

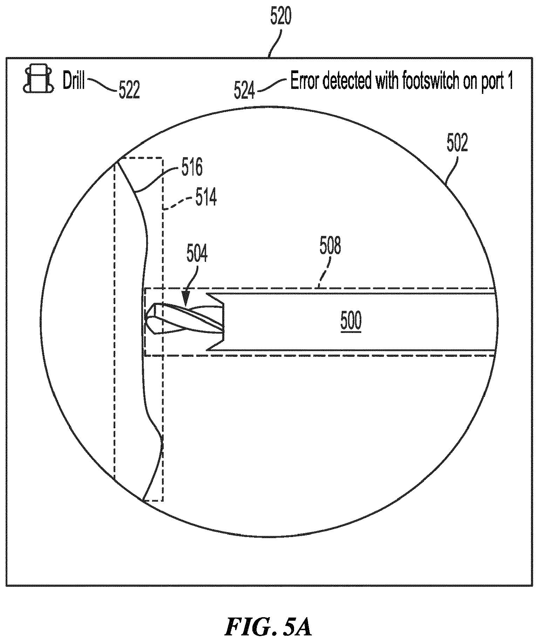

The surgical tool module 258 can be configured to determine the presence of one or more surgical tools in a given image frame. Certain surgical tools can be associated with different available functionalities according to which the surgical tool may operate. Thus, in one or more examples, surgical tool module 258 can include one or more classifiers configured to determine the presence (or absence) of various tools in the surgical environment that have various available functionalities, which can include determining a type, or class, of a tool in the surgical environment. For example, the one or more classifiers can be configured to determine the presence or absence of a cutter, a bur, an RF probe, a drill, and/or any other surgical tool that has a given type of functionality. Types of functionalities may include, for example, a suction functionality, a resection functionality, a wash functionality, a cutting functionality, a drilling functionality, a high-power functionality, a low-power functionality, a high-RPM functionality, a low-RPM functionality, an oscillation functionality, a forward drilling functionality, and/or a backward drilling functionality. In one or more examples, the surgical tool module 258 can include multiple classifiers, each classifier configured to determine the presence of one or more types of surgical tools, or can include a single classifier that can determine the presence of multiple types of surgical tools. For instance, surgical tool module 258 can include a first classifier configured to determine if a cutter is present in the surgical environment and a second classifier configured to determine if a bur is present in the surgical environment, or the surgical tool module 258 can include a single classifier that can determine if a cutter and a bur is present in the surgical environment. FIG. 5A illustrates an example of the surgical tool module 258 detecting the presence of a surgical tool 500 in an endoscopic video frame 502. The surgical tool module 258 may detect the presence of surgical tool 500 and may determine that the type of the surgical tool 500 is a drill, such as based on the unique visual characteristics of the drill bit tip 504 of the drill relative to other types of surgical tool 500 that the surgical tool module 258 is configured to detect. The surgical tool module 258 may detect the presence of the surgical tool 500 and, optionally, determine the location of the surgical tool 500 within the frame 502, as represented by the bounding box 508.

In one or more examples, the surgical tool module 258 can be configured to determine the presence of a tool or portion of a tool that is outside of a surgical cavity. For example, the surgical tool module 258 can be configured to determine the presence of a shaver in one or more image frames. Because a shaver (to which a rotating tool, such as a bur or cutter, is connected) is not typically operated within the surgical cavity, the presence of the shaver in the one or more image frames may indicate that the endoscopic imaging device has been removed from the surgical cavity. As such, classification output data from the surgical tool module 258 indicating that a shaver is in one or more image frames may be a trigger used (e.g., by surgical pump 262) to reduce or shut off one or more functionalities of one or more other surgical tools that are determined not to be in the surgical cavity and/or to unpair the surgical input device from the one or more other surgical tools that are determined not to be in the surgical cavity. In one or more examples, the surgical tool module 258 can be configured to determine a type of surgical tool located outside of the surgical cavity. Such a determination may be used to enable or disable functionality of the identified tool type and/or to unpair a surgical input device from the identified tool type.

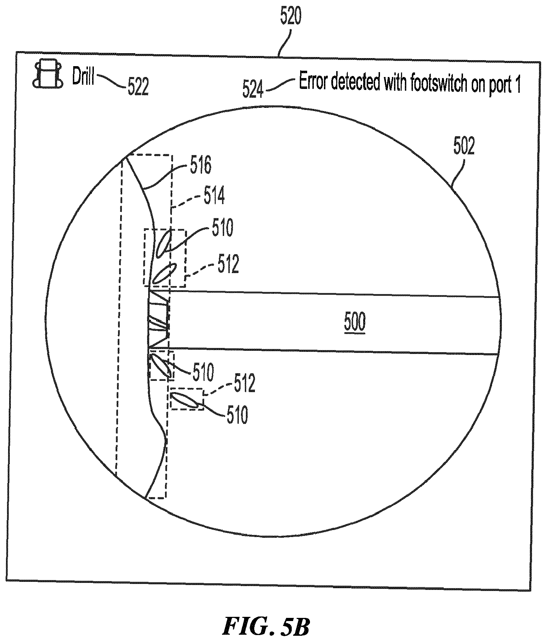

Image quality module 256 can be configured to determine the presence of various features associated with the clarity of one or more image frames. As described above, and described in detail below, various conditions that can inhibit the clarity of a video, such as blood, bubbles, debris, snow globe conditions, and turbidity can be mitigated by changing one or more settings of surgical pump 262 output, such as the pressure, inflow flow rate, and/or suction flow rate. Thus, in one or more examples, image quality module 256 includes one or more classifiers configured to generate classification output data indicating the presence in one or more image frames of one or more of blood, bubbles, debris, snow globe conditions, and/or turbidity. An example of the detection by image quality module 256 of the presence of various features associated with the clarity of one or more image frames is illustrated in FIG. 5B. The frame 502 captures debris 510 being generated during use of the surgical tool 500 (e.g., a drill) on bone 516. The image quality module 256 is configured to detect debris 510 in the frame 502, as indicated in FIG. 5B by bounding boxes 512. Each feature relating to clarity can be determined by its own classifier or multiple features relating to clarity can be determined by a single classifier. In one or more examples, one or more classifiers can be configured to determine a degree of presence of a given feature. For example, a classifier for determining the presence of blood may determine three classes associated with blood-no blood, a relatively small amount of blood, and a relatively large amount of blood. These classes are merely exemplary, and it will be understood by one of skill in the art that any number of classes may be used and that the number of classes may differ for different features.

Anatomical object module 260 can include one or more classifiers configured for determining the presence of one or more anatomical objects in a given image frame. Anatomical objects may include, for example, body parts and/or implants that are to be inserted or have been inserted into a body. Certain anatomical objects can be associated with different surgical tools and/or different surgical tool functionalities that may be used to operate on or otherwise interact with the anatomical object. Thus, in one or more examples, anatomical object module 260 can include one or more classifiers configured to determine the presence (or absence) of various anatomical objects in the surgical environment that may be operated on or may interact with various available tools and/or tool functionalities; this may include determining a type of anatomical object in the surgical environment. For example, the one or more classifiers can be configured to determine the presence or absence of a tissue, muscle, bone, blood, an implant, and/or any other anatomical object that interacts with a given surgical tool or a given tool functionality. Types of functionalities may include, for example, a suction functionality, a resection functionality, a wash functionality, a cutting functionality, a drilling functionality, a high-power functionality, a low-power functionality, a high-RPM functionality, a low-RPM functionality, an oscillation functionality, a forward drilling functionality, and/or a backward drilling functionality. In one or more examples, the anatomical object module 260 can include multiple classifiers, each classifier configured to determine the presence of one or more types of anatomical objects, or can include a single classifier that can determine the presence of multiple types of anatomical objects. For instance, the anatomical object module 260 can include a first classifier configured to determine if bone is present in the surgical environment and a second classifier configured to determine if muscle is present in the surgical environment, or anatomical object module 260 can include a single classifier that can determine if bone and muscle is present in the surgical environment. For example, with reference to FIGS. 5B, the anatomical object module 260 may determine the presence of the bone 516 in the frame 502, as indicated by bounding box 514.

In some aspects, one or more additional or alternative classifiers may be included in image processing system 250. The one or more additional or alternative classifiers may be included in one or more of the same modules as described above with respect to image processing system 250, stored in classifier data store 206, and/or they may be included in one or more additional or alternative modules. For example, in some aspects image processing system 250 may comprise a tool location classifier configured to generate classification output data identifying a location of a surgical tool, an anatomical position classifier configured to generate classification output data identifying a position of an anatomical structure, a surgery-type classifier configured to generate classification output data identifying a surgery type, a surgery-stage classifier configured to generate classification output data identifying a surgery stage, and/or an implant location classifier configured to generate classification output data identifying a location of an implant.

The image processing system 250 can transmit the results of the feature determinations performed by the one or more modules to a controller 264 of surgical pump 262 for the controller 264 to determine if any adjustments to pairings, surgical tool functionality settings, and/or input device modality settings are required in light of the classification output data generated by the one or more classifiers. Surgical pump 262 may share any one or more characteristics in common with surgical pump 120 of FIG. 1. In one or more examples, controller 264 can receive classification output data (e.g., including inference values) from each of the classifiers and make a determination regarding adjustments to pairings, surgical tool functionality settings, and/or input device modality settings based on the output of one or more of the classifiers. In this way, controller 264 can make decisions as to pairing settings, surgical tool functionality settings, and/or input device modality settings at any given moment during a surgery based on a plurality of conditions that may occur during a surgery.

In one or more examples, determinations regarding adjustments to pairings, surgical tool functionality settings, and/or input device modality settings are made by image processing system 250 (rather than by controller 264) based on the features determined to be present in the surgical field. The image processing system 250 may then provide instructions to controller 264.

Regardless of which component determines adjustments to be made to pairings, surgical tool functionality settings, and/or input device modality settings, instructions to execute said adjustments may be transmitted to the affected surgical tools 208 and/or surgical input device 210.

In one or more examples, one or more functions of image processing system 250 described above can be performed by the surgical pump 262 and/or by another system component. For example, the surgical pump 262, an input device, and/or a surgical tool may implement one or more of the image quality module 256, the surgical tool module 258, and the anatomical object module 260.

In one or more examples, the image processing system 250 or the surgical pump 262 implements a machine learning model controller that is configured to take as input one or more image frames and determine as an output one or more adjustments to be made to pairings between an input device and surgical tools, surgical tool functionality settings, and/or input device modality settings. For example, the image processing system 250 or the surgical pump 262 can implement a deep neural network (DNN) controller generated using model-free reinforcement learning. In one or more examples, the DNN controller can be trained using a trained DNN-based simulator that generates simulated training data sets on which the DNN controller can be trained. The DNN simulator is trained using training data from a physical system, either a physical system operating in real-world scenarios (such as input devices and/or surgical tools used during a surgical procedure) or a physical system used in a prototyped environment (such as input devices and/or surgical tools used in a prototyped surgical environment). In one or more examples, the machine learning model controller uses inputs other than image frames to determine pairing adjustments, surgical tool functionality settings, and/or input device modality settings. Examples of inputs that may be used are data from sensors that measure one or more tool characteristics (e.g., pressure, speed, temperature, and/or flow rate) in the surgical environment and/or data from one or more patient monitor devices (e.g., measuring blood pressure, heart rate, temperature, etc.).

The classifiers of the one or more modules of the image processing system 250 can be trained using a supervised training process, an unsupervised training process, a self-supervised training process, a weakly-supervised training process, or a semi-supervised training process. Different classifiers can be trained using a different training process. In a supervised training process, the classifier can be trained by using training images. Each training image can be annotated, for example, by appending metadata to the image that identifies one or more characteristics of the image. For instance, using a hip joint classifier configured to identify the presence of a hip joint in an image as an example, the classifier can be trained using a plurality of training images known *(a priori)* to visualize hip joints.

As described herein, classification output data from one or more of the classifiers may be used by system 200 to automatically update pairings between surgical input device 210 and surgical tools 208, for example by pairing surgical input device 210 to a new one of surgical tools 208, unpairing surgical input device 210 from one of surgical tools 208, and/or switching surgical input device 210 from being paired to one of surgical tools 208 to being paired to another one of surgical tools 208. As described herein, classification output data from one or more of the classifiers may be used by system 200 to automatically set or update a functionality for one or more of surgical tools 208. As described herein, classification output data from one or more of the classifiers may be used by system 200 to automatically set or update a modality for surgical input device 210.

While the description herein primarily describes use of classifiers to generate classification output data, any other suitable kind of data processing algorithm may be used to generate output data based on the one or more images, and the output data may subsequently be used in a same or similar manner as described with respect to classifiers. For example, a computer vision algorithm may be used to generate output data based on input data comprising one or more images, and the generated output data may be used as described herein in a same or similar manner as classification output data generated by a classifier.

In a further example, system 200 may be configured to use the classification output data to anticipate switches between pairings (e.g., switching surgical input device 210 from being paired with a first surgical tool to a second surgical tool), functionality settings (e.g., switching a surgical tool from a first functionality to a second functionality), and/or modality settings (e.g., switching surgical input device 210 from a first modality to a second modality). A system configured to anticipate that a user is going to request a switch may generate classification output data indicating a probability that a specified device pairing switch will occur within a certain time window. If a user request to switch can be anticipated in this manner (even if only by a few seconds) a voice command mode may be activated ahead of time and may allow for efficient transitions between modalities, for example by automatically activating a microphone and/or automatically activating a listening mode allowing a user to access a voice command functionality usable to switch modes without requiring the user to manually activate a microphone or manually use a voice-command trigger phrase to activate a listening mode. Additionally or alternatively, if switches can be anticipated (even if only by a few seconds) visualization of a suggested switch may be displayed ahead of time and may allow for efficient transitions between modalities, for example by allowing a user to view and confirm the suggested switch without needing to manually select and execute the suggested switch.

User preference data source 212 may include any computer-readable storage medium storing user preference data for one or more users (e.g., surgeons, medical staff, and the like). User preference data source 212 may store one or more user profiles indicating user preferences for certain users, which may for example indicate preferred functionalities and/or preferred modalities that are associated with certain users, for example by ranking preferred functionalities/modalities and/or by indicating certain functionalities/modalities that the specific user may desire to use or may desire to avoid using. User preference data may optionally be used by image processing system 250 to select a functionality for an identified surgical tool, and/or to select a modality for an input device configured to be used with the identified surgical tool.

User input device 214 may be communicatively coupled to image processing system 250 and configured to receive a user input that is usable by image processing system 250 to identify a surgical tool from surgical tools 208, to select a functionality for an identified surgical tool, and/or to select a modality for user input device 214. User input device 214 may include one or more keyboards, mouses, touch-screens, touch-sensitive devices, microphones, buttons, keys, knobs, dials, switches, pedals, pointing devices, and/or the like.

An input received from user input device 214 may be a user input received before an identification and/or selection is made by image processing system 250 and may serve as a basis (in whole or in part) for the identification and/or selection made by image processing system 250. An input received from user input device 214 may include a confirmatory user input received after an identification and/or selection is made by image processing system 250, and image processing system 250 may use the confirmatory user input as a basis (in whole or in part) for confirming the identification and/or selection made.

While user input device 214 is shown in FIG. 2 as separate from other components of system 200, user input device 214 may be provided as part of (e.g., included in the same housing) as one or more other components of system 200. For example, user input device 214 may comprise buttons on one of surgical tools 208 and/or may comprise buttons on surgical input device 210.

Display device 216 may be communicatively coupled to image processing system 250 and configured to display one or more images captured by image capture device 204, information regarding processing of one or more images by image processing system 250, information regarding one or more of surgical tools 208 paired with surgical input device 210, information regarding a selected or suggested functionality (e.g., a functionality suggested by system 200 but not implemented by system 200 without user confirmation) for one or more of surgical tools 208, and/or information regarding a selected or suggested modality (e.g., a modality suggested by system 200 but not implemented by system 200 without user confirmation) for surgical input device 210. Display device 216 may display suggested actions (e.g., pairing surgical input device 210 to a surgical tool, setting a surgical tool to a suggested functionality, and/or setting surgical input device 210 to a suggested modality) which may be executed upon receiving a confirmatory user input via user input device 214.

Optionally, system 200 may display one or more suggested pairings and/or settings to a user, for example by determining how many suggestions to display in accordance with a confidence level of a determination made by system 200. In some examples, a confidence level may be determined based on (e.g., may be proportional to) classification output data (e.g., including inference values) generated by one or more of the classifiers, such that a confidence level for an action suggested based on a determined condition present in the surgical environment may be proportional to a degree of certainty (based on the classification output data) with which the system has determined the condition to be present. A list of suggestions including text and/or graphical representation may be displayed for the user (e.g., via display device 216).

Optionally, a confirmatory user input may be provided by an audible confirmation. When options are displayed to the user, system 200 may activate a voice command mode. System 200 may activate a microphone to detect a voice command from the user. If no voice command is detected in a predetermined amount of time, the suggestion may time out and may cease to be displayed. In the event of a timeout of the suggestion, no action may be taken, or a top suggestion (e.g., a suggestion having a highest confidence level as determined based on the classification output data) may be automatically executed by system 200.

Optionally, even in the event that system 200 does not suggest any new pairing or setting, a user may still be able to execute a voice command, for example by uttering a trigger phrase and then uttering a voice command. System 200 may then display and/or audibly output its perceived command, prompting the user to confirm the perceived command with a confirmatory user input executed using user input device 214 and/or using a further voice command.

FIG. 3 depicts a method 300 for automatically configuring a surgical tool and an input device for the surgical tool. Method 300 may be performed by a system comprising a plurality of surgical tools, a user input device, a surgical input device, one or more processors, and a computer-readable storage medium storing instructions executable by the one or more processors to cause the system to perform method 300. Method 300 may be performed by system 200, and is described below with reference to system 200.

At block 302, system 200 may receive one or more images. Image processing system 250 of system 200 may receive the one or more images. The one or more images may be received by image processing system 250 by from image capture device 204. The one or more images may be received in real time (e.g., as a series of still images, streaming video data, and the like). The one or more images may be received from any other suitable source, including from a source (e.g., a storage medium, computer system, and/or video capture device) internal or external to system 200.

After receiving (and/or while receiving) the one or more images, system 200 may apply one or more image processing techniques (e.g., video processing techniques) to the received one or more images.

At block 304, system 200 may apply one or more classifiers to the one or more images received to generate classification output data. The one or more classifiers applied may include a tool identification classifier configured to generate classification output data identifying a surgical tool, a tool location classifier configured to generate classification output data identifying a location of a surgical tool, an anatomical identification classifier configured to generate classification output data identifying an anatomical feature, an anatomical position classifier configured to generate classification output data identifying a position of an anatomical structure, a surgery-type classifier configured to generate classification output data identifying a surgery type, a surgery-stage classifier configured to generate classification output data identifying a surgery stage, an implant classifier configured to generate classification output data identifying an implant, an implant location classifier configured to generate classification output data identifying a location of an implant, and/or any one or more of the classifiers described with respect to image quality module 256, surgical tool module 258, and/or anatomical object module 260. The classification output data generated by the one or more classifiers may comprise one or more inference values. Additionally or alternatively, an object detection algorithm may be used to generate an output specifying both an object type and a location of the obj ect with respect to an internal portion of a patient. The output generated by an object detection algorithm may be used in addition to or alternatively to the classification output data described above or described elsewhere herein, and may be used in any manner in which classification output data may be used as described herein. In some examples, object detection algorithms may be provided by Region-based Convolutional Neural Network (R-CNN) architectures and/or by You Only Look Once (YOLO) network architectures.

Optionally, a classifier can be trained by directly labeling training images and/or video fragments with corresponding control device modes. This multi-label classification framework may provide a classifier that outputs probabilities of desired pairings for surgical input devices and surgical tools. Optionally, a single probability may be assigned to a full set of configurations, wherein the full set of configurations may include one or more of the following configurations: a pairing of surgical input device 210 with an identified surgical tool, a setting of a functionality for the identified surgical tool, and a setting of a modality for surgical input device 210. Optionally, individual probabilities may be assigned to individual configurations.

The classification output data generated by the one or more classifiers may be stored locally, transmitted to one or more components of system 200, transmitted to one or more systems aside from system 200, used to trigger one or more automated system functionalities, used to trigger one or more alerts, displayed (e.g., on display device 216), and/or used to generate one or more visualizations.

Optionally, system 200 may analyze the received one or more images in any additional suitable manner, for example by applying one or more data processing operations to the received one or more images to generate classification output data that may be used as a basis for subsequent programmatic logic. Optionally, analysis of the one or more images (e.g., using AI and/or ML) may be used to detect surgical tools that are present in the one or more images, to detect anatomical features that are present in the one or more images, to detect implants and/or prosthetics that are present in the one or more images, to determine joint condition of a j oint that is present in the one or more images, to determine a position of an anatomical structure that is present in the one or more images, to determine an anatomical structure that is present in the one or more images, to determine a surgery type of a surgical procedure that is shown in whole or in part in the one or more images, and/or to determine a surgery stage of a surgical procedure that is shown in whole or in part in the one or more images.

As an example of block 304 and with reference to FIGS. 5A, a tool identification classifier may generate classification output data associated with a prediction that the surgical tool 500 in the frame 502 is a drill (e.g., a percentage likelihood that the surgical tool 500 is a drill and may also calculate one or more percentage likelihoods that the surgical tool 500 is one or more other types of surgical tools). An anatomical identification classifier may generate classification output data associated with a prediction that the frame 502 includes the bone 516.

At block 310, system 200 may identify a surgical tool from the set of surgical tools 208. For example, with reference to FIG. 5A, the system may identify the surgical tool 500 as a drill. The identification of the surgical tool may be based, in whole or in part, on the classification output data generated at block 304. For example, image processing system 250 may identify a surgical tool from amongst surgical tools 208 by applying a tool identification classifier. Identification of the surgical tool may optionally be based on a determined tool location, a determined anatomical identification, a determined position of an anatomical structure, a determined surgery type, and/or a determined surgery stage. Classification output data (including combinations of classification output data generated by different classifiers) may be mapped to any one or more surgical tools. For example, one or more predefined rules stored in a database or otherwise accessible by system 200 may be applied to identify one or more surgical tools in accordance with the system determining that a set of one or more associated classification outputs have been generated.

The identified surgical tool may be automatically configured by the system in any suitable manner, including for being paired with surgical input device 210.

At block 314, system 200 may pair surgical input device 210 with the identified surgical tool. For example, with reference to FIG. 5A, the system may pair the surgical input device 210 with the surgical tool 500. Pairing surgical input device 210 with the identified surgical tool may comprise configuring one or both of surgical input device 210 and the identified surgical tool for direct or indirect wireless communication with one another, for example by a network communication handshake to be executed between them. Surgical input device 210 and the identified surgical tool may be paired with one another for direct wireless communication and/or for indirect wireless communication, for example as facilitated by image processing system 250 and/or one or more additional components of system 200. By being paired with one another, surgical input device 210 and the identified surgical tool may be configured such that surgical input device 210 may be used to operate the identified surgical tool.

Pairing surgical input device 210 with the identified surgical tool may be controlled by an algorithm executed by surgical pump 262 (e.g., by controller 264 thereof). The algorithm may include determining a current status of surgical input device 210 (e.g., indicating to which surgical tool surgical input device 210 is paired), referencing one or more inference values generated as classification output data by the one or more classifiers, and determining on the basis of the current status and the inference values whether the current status of surgical input device 210 should be changed to pair it with the identified surgical tool. Optionally, pairing surgical input device 210 with the identified surgical tool may comprise transmitting an instruction for the pairing from image processing system 250 to one or both of surgical input device 210 and the identified surgical tool.

Surgical pump 262 may receive identification data comprising (or generated based on) the classification output data indicating the identified surgical tool. For example, a connected OR hub or other edge computing device may execute the one or more classifiers to generate the classification output data, and may then transmit the generated classification output data to surgical pump 262, for example by transmitting the generated classification output data to surgical pump 262 via SFB.

Surgical pump 262 may then determine a current status of surgical input device 210, for example by looking up a stored data value indicating a current status of surgical input device 210, which may be stored in computer memory provided as part of surgical input device 210, as part of surgical pump 262, as part of the OR hub or other edge device, or elsewhere in system 200. Additionally or alternatively, surgical pump 262 may determine the current status of surgical input device 210 by querying surgical input device 210 with a transmission requesting a status update to be sent from surgical input device 210 to surgical pump 262. The current status of surgical input device 210 may include information indicating a pairing status of surgical input device 210, for example by indicating to which surgical tool (if any) surgical input device 210 is paired. Additionally, the current status of surgical input device 210 may include information indicating a toggle position of surgical input device 210, for example by indicating a current state of mappings from certain buttons, pedals, or the like of surgical input device 210 to respective functionalities of a given surgical tool.

Surgical pump 262 may then determine whether the current status of surgical input device 210 should be updated based on the received identification data comprising the classification output data indicating the identified surgical tool. For example, the one or more processors executing the algorithm may access stored data, such as a rules database, storing rules associating certain classification output data with certain input device statuses, and may check whether the current status of surgical input device 210 is in compliance with the accessed rules. In one example, the classification output data may identify a surgical tool that is in a field of view of an endoscope that captured the one or more images, and the algorithm may check whether the current status of surgical input device 210 indicates that surgical input device 210 is paired to the identified surgical tool.

If it is determined that the current status of surgical input device 210 and the classification output data are in compliance with the accessed rules, then no action may be taken. If it is determined that the current status of surgical input device 210 and the classification output data are not in compliance with the accessed rules, then the one or more processors executing the algorithm may cause the current status of surgical input device 210 to be updated to comply with the accessed rules. For example, the one or more processors executing the algorithm may cause the current status of surgical input device 210 to be updated to pair surgical input device 210 with the surgical tool identified in the field of view of the endoscope.

In order to cause the current status of surgical input device 210 to be updated, surgical pump 262 may send a command to surgical input device 210 (and/or to a controller thereof) to cause the current status of surgical input device 210 to be updated to pair surgical input device 210 to the identified surgical tool. Surgical input device 210 (and/or a controller thereof) may receive the command and may responsively update a stored data value to cause an update to the current status (e.g., pairing status) of surgical input device 210.

Optionally, system 200 may cause an indication to be displayed (e.g., via display device 216) indicating that surgical input device 210 and the identified surgical tool have been paired with one another.

At block 316, system 200 may select a functionality for the identified surgical tool. The selection of the functionality may be based, in whole or in part, on the classification output data generated at block 304. For example, image processing system 250 may select a functionality for the identified surgical tool based on a determined tool identification, a determined tool location, a determined anatomical identification, a determined position of an anatomical structure, a determined surgery type, and/or a determined surgery stage. For example, a rules database or other stored data may map any combination of classification output data to one or more functionalities, and the system may determine which functionality or functionalities to select based on which classification output data has been generated. The selected functionality may be based on which surgical tool was identified at block 310, for example in instances in which a rules database or other stored data may indicate that only certain functionalities are available for use with certain surgical tools.

At block 318, system 200 may set the identified surgical tool to the selected functionality that was selected at block 316. Setting the identified surgical tool to the selected functionality may comprise configuring the identified surgical tool to function in accordance with the selected functionality, such as by operating in any one or more of a plurality of modes.

In some aspects, a system leveraging the techniques disclosed herein may be configured to set a resection device (e.g., a bursector) to one of a plurality of functionalities, for example in accordance with which anatomical structure is detected as being resected based on one or more images. For example, if bone is detected, such as illustrated in FIG. 5A and 5B (bone 516), a resection device, such as surgical tool 500, may be set to a forward drilling functionality and/or to a high RPM functionality; if tissue is detected, the resection device may be set to an oscillation functionality and/or to a lower RPM functionality. Determination (e.g., at block 316) as to which resection device functionality should be set may be made based in part on joint space and anatomy that is being operated on, as indicated by the classification output data (e.g., generated by the one or more classifiers of anatomical object module 260 shown in FIG. 2).

In some aspects, a system leveraging the techniques disclosed herein may be configured to set an RF probe device for either a resection functionality or a coagulation functionality, for example in accordance with whether bleeding is detected in the received one or more images. In some aspects, a system leveraging the techniques disclosed herein may be configured to set an RF probe device to different functionalities corresponding to different power levels in accordance with whether meniscus is visible in one or more images (e.g., video data) and/or in accordance with whether one or more images (e.g., video data) indicates that a user (e.g., surgeon) is operating on meniscus. Determination (e.g., at block 316) to set the RF probe functionality to the "coagulate mode" may be made based in part on determining that blood is detected in a joint and that the RF probe is inserted, as indicated by the classification output data (e.g., generated by the one or more classifiers of image quality module 256 and surgical tool module 258, respectively).

In some aspects, a system leveraging the techniques disclosed herein may be configured to set a sinuscope to either a rinse functionality or to a suction functionality based on visibility in one or more images and/or based on various conditions (e.g., blood, bubbles, debris, snow globe conditions, turbidity, and the like) detected in a sinus cavity visible in the one or more images. In some aspects, a sinuscope may operate according to a plurality of different functionalities, where one functionality corresponds to a pulse functionality for pulsing fluid and another functionality corresponds to a suction functionality. Determination (e.g., at block 316) to set the sinuscope to a pulse functionality may be made based on detection of one or more of the various conditions that can inhibit the clarity of the one or more images (to pulse fluid into the sinus cavity), as indicated by the classification output data (e.g., generated by the one or more classifiers of image quality module 258).

In some aspects, a system leveraging the techniques disclosed herein may be configured to set an implant insertion device to one or more functionalities based on what implant is detected based on one or more images and/or based on what step in an implant process is detected based on one or more images.

In some aspects, a system leveraging the techniques disclosed herein may be configured to change a pairing of surgical input device 210 and/or set a surgical tool to one of various functionalities for drilling, cutting bone, and/or screwing in an implant.

Setting the identified surgical tool to the selected functionality may include determining a current status of the identified surgical tool (e.g., indicating to which functionality the identified surgical tool is currently set), referencing one or more inference values generated as classification output data by the one or more classifiers, and determining on the basis of the current status and the classification output data whether the current status of the identified surgical tool should be changed to be set to the selected functionality. Optionally, setting the identified surgical tool to the selected functionality may comprise transmitting an instruction for the setting from image processing system 250 to the identified surgical tool.

Setting the identified surgical tool to the selected functionality may be controlled by an algorithm executed by surgical pump 262 (e.g., by controller 264 thereof). The algorithm may include determining a current status of the identified surgical tool (e.g., indicating to which functionality the identified surgical tool is currently set), referencing one or more inference values generated as classification output data by the one or more classifiers, and determining on the basis of the current status and the classification output data whether the current status of the identified surgical tool should be changed to set it to the selected functionality. Optionally, setting the identified surgical tool to the selected functionality may comprise transmitting an instruction for the setting from image processing system 250 to one or both of surgical input device 210 and the identified surgical tool.

Surgical pump 262 may receive selection data comprising (or generated based on) the classification output data indicating the selected functionality. For example, a connected OR hub or other edge computing device may execute the one or more classifiers to generate the classification output data, and may then transmit the generated classification output data to surgical pump 262, for example by transmitting the generated classification output data to surgical pump 262 via SFB.

Surgical pump 262 may then determine a current status of the identified surgical tool, for example by looking up a stored data value indicating a current functionality of the identified surgical tool, which may be stored in computer memory provided as part of surgical input device 210, as part of the identified surgical tool, as part of surgical pump 262, as part of the OR hub or other edge device, or elsewhere in system 200. Additionally or alternatively, surgical pump 262 may determine the current status of the identified surgical tool by querying the identified surgical tool with a transmission requesting a status update to be sent from the identified surgical tool to surgical pump 262. The current status of the identified surgical tool may include information indicating a current functionality to which the identified surgical tool is set.

Surgical pump 262 may then determine whether the current status of the identified surgical tool should be updated based on the received selection data comprising the classification output data indicating the selected functionality. For example, the one or more processors executing the algorithm may access stored data, such as a rules database, storing rules associating certain classification output data with certain surgical tool statuses, and may check whether the current status of the identified surgical tool is in compliance with the accessed rules.

If it is determined that the current status of the identified surgical tool and the classification output data are in compliance with the accessed rules, then no action may be taken. If it is determined that the current status of the identified surgical tool and the classification output data are not in compliance with the accessed rules, then the one or more processors executing the algorithm may cause the current status of the identified surgical tool to be updated to comply with the accessed rules. For example, the one or more processors executing the algorithm may cause the current status of the identified surgical tool to be updated to set the identified surgical tool to the selected functionality.

In order to cause the current status of the identified surgical tool to be updated, surgical pump 262 may send a command to the identified surgical tool to cause the current status of the identified surgical tool to be updated to set the identified surgical tool to the selected functionality. The identified surgical tool may receive the command and may responsively update a stored data value to cause an update to the current status (e.g., the functionality) of the identified surgical tool. Optionally, the command may be sent to surgical input device 210 or to another component of system 200, which may execute the command or may forward the command to the identified surgical tool.

Optionally, system 200 may cause an indication to be displayed (e.g., via display device 216) indicating that the identified surgical tool has been set to the selected functionality.

At block 320, system 200 may select a modality for surgical input device 210. The selection of the modality may be based, in whole or in part, on the classification output data generated by image processing system 250 at block 304. For example, image processing system 250 may select a modality for surgical input device 210 based on a determined tool identification, a determined tool location, a determined anatomical identification, a determined position of an anatomical structure, a determined surgery type, and/or a determined surgery stage. For example, a rules database or other stored data may indicate mappings for any combination of classification output data to one or more modalities, and the system may use the mappings to determine which modality or modalities to select based on which classification output data has been generated. The selected modality may optionally be based on which surgical tool was identified at block 310 and/or on which functionality was selected at block 316, for example in instances in which a rules database or other stored data may indicate that only certain modalities are available for use with certain functionalities and/or with certain surgical tools. For example, and with reference to FIG. 5A, system 200 may select a drill control modality for surgical input device 210 based on detecting the presence of the surgical tool 500 and detecting the identity of the surgical tool 500 as a drill. The drill control modality for the surgical input device 210 could include a mapping of a first input of the surgical input device 210 (e.g., a first button of a footswitch) to control a speed of rotation of the drill and a second input of the surgical input device 210 (e.g., a second button of a footswitch) to control a direction of rotation of the drill.

At block 322, system 200 may set surgical input device 210 to the selected modality that was selected at block 320. Setting surgical input device 210 to the selected modality may comprise configuring surgical input device 210 such that one or more components of surgical input device 210 are mapped to one or more functionalities of the identified surgical tool that was selected at block 316. For example, setting a modality of surgical input device 210 may comprise mapping certain buttons, pedals, or the like of surgical input device 210 to certain functionalities of the identified surgical tool. Setting a modality of surgical input device 210 may include, for example, setting surgical input device 210 to a right-handed modality or to a left-handed modality, wherein the mapping of certain components of surgical input device 210 to certain functionalities (e.g., forward drilling and reverse drilling functionalities) of the identified surgical tool are reversed with respect to one another when surgical input device 210 is set to the right-handed modality versus the left-handed modality.

In some aspects, setting a modality of surgical input device 210 may include automatically changing which foot pedals of a foot pedal device are mapped to which functionalities of a surgical tool. For example, setting a modality of surgical input device 210 may comprise modifying which foot pedal is mapped to a start/stop functionality of a pump and which foot pedal is mapped to a wash functionality. In one example, system 200 may set surgical input device 210 to a modality to control start/stop functionality of a pump if blood is not detected in a joint (based on classification output data of the classifiers) while system 200 may set surgical input device 210 to a modality to control a wash functionality of the pump if blood is detected in the joint (based on classification output data of the classifiers).

Setting surgical input device 210 to the selected modality may include determining a current status of the identified surgical tool (e.g., indicating to which modality the identified surgical tool is currently set), referencing one or more inference values generated as output by the one or more classifiers, and determining on the basis of the current status and the inference values whether the current status of surgical input device 210 should be changed to be set to the selected modality.

Setting surgical input device 210 to the selected modality may be controlled by an algorithm executed by surgical pump 262 (e.g., by controller 264 thereof). The algorithm may include determining a current status of surgical input device 210 (e.g., indicating to which modality surgical input device 210 is currently set), referencing one or more inference values generated as classification output data by the one or more classifiers, and determining on the basis of the current status and the classification output data whether the current status of surgical input device 210 should be changed to set it to the selected modality. Optionally, setting surgical input device 210 to the selected modality may comprise transmitting an instruction for the setting from image processing system 250 to one or both of surgical input device 210 and the identified surgical tool.

Surgical pump 262 may receive selection data comprising (or generated based on) the classification output data indicating the selected modality. For example, a connected OR hub or other edge computing device may execute the one or more classifiers to generate the classification output data, and may then transmit the generated classification output data to surgical pump 262, for example by transmitting the generated classification output data to surgical pump 262 via SFB.

Surgical pump 262 may then determine a current status of surgical input device 210, for example by looking up a stored data value indicating a current modality of surgical input device 210, which may be stored in computer memory provided as part of surgical input device 210, as part of the identified surgical tool, as part of surgical pump 262, as part of the OR hub or other edge device, or elsewhere in system 200. Additionally or alternatively, surgical pump 262 may determine the current status of surgical input device 210 by querying surgical input device 210 with a transmission requesting a status update to be sent from surgical input device 210 to surgical pump 262. The current status of surgical input device 210 may include information indicating a current modality to which surgical input device 210 is set, for example by indicating a current toggle position of surgical input device 210. As described herein, a toggle position of surgical input device 210 may indicate a current state of mappings from certain buttons, pedals, or the like of surgical input device 210 to respective functionalities of a given surgical tool of the set of surgical tools. For example, a first toggle position of surgical input device 210 may correspond to mappings from certain buttons, pedals, or the like of surgical input device 210 to various functionalities of a shaver hand piece. Similarly, a second toggle position of surgical input device 210 may correspond to mappings from certain buttons, pedals, or the like of surgical input device 210 to various functionalities of an RF probe.

Surgical pump 262 may then determine whether the current status of surgical input device 210 should be updated based on the received selection data comprising the classification output data indicating the selected modality. For example, the one or more processors executing the algorithm may access stored data, such as a rules database, storing rules associating certain classification output data with certain surgical input device statuses, and may check whether the current status of surgical input device 210 is in compliance with the accessed rules. In one example, the classification output data may identify a surgical tool that is in a field of view of an endoscope that captured the one or more images, and the algorithm may check whether a current toggle position of surgical input device 210 should be modified based on the classification output data and based on whether the selected modality is compatible with the current toggle position. In one example, the algorithm may determine that a current toggle position should be modified if the identified surgical tool is not compatible with a current toggle position of surgical input device 210.

If it is determined that the current status of surgical input device 210 and the classification output data are in compliance with the accessed rules, then no action may be taken. If it is determined that the current status of surgical input device 210 and the classification output data are not in compliance with the accessed rules, then the one or more processors executing the algorithm may cause the current status of surgical input device 210 to be updated to comply with the accessed rules. For example, the one or more processors executing the algorithm may cause the current status of surgical input device 210 to be updated to set surgical input device 210 to the selected modality.

In order to cause the current status of surgical input device 210 to be updated, surgical pump 262 may send a command to surgical input device 210 (and/or to a controller thereof) to cause the current status of surgical input device 210 to be updated to set surgical input device 210 to the selected modality. Surgical input device 210 (and/or a controller thereof) may receive the command and may responsively update a stored data value to cause an update to the current status (e.g., modality) of surgical input device 210.

Optionally, setting surgical input device 210 to the selected modality may comprise transmitting an instruction for the setting from image processing system 250 to surgical input device 210.

Optionally, system 200 may cause an indication to be displayed (e.g., via display device 216) indicating that surgical input device 210 has been set to the selected modality.

As shown at block 306, system 200 may optionally receive a user input and use the user input as part of the basis for identifying a surgical tool at block 310, selecting a functionality for the identified surgical tool at block 316, and/or selecting a modality for surgical input device 210 at block 320. The received user input may comprise an instruction to select a surgical tool, to select a functionality, and/or to select a modality. The received user input may be received before one or more of the identifications/selections at blocks 310, 316, and 320 are made. The user input received at block 306 may be received via user input device 214, via surgical input device 210, and/or via any other input device.

As shown at block 308, system 200 may optionally receive user preference data and use the user preference data as part of the basis for identifying a surgical tool at block 310, selecting a functionality for the identified surgical tool at block 316, and/or selecting a modality for surgical input device 210 at block 320. The received user preference data may comprise an explicit indication (e.g., as input and stored by a user) of a user preference and/or may comprise historical information regarding prior surgical tools used, prior functionalities used, and/or prior modalities used by a user. In some instances, for example when neither a user's explicit input nor the user's historical data is available (e.g., a new user who does not have accessible profile information), the system may set default preferences for the user based on historical values for one or more other users. For example, historical preferences that are most common for a surgery of a given type may be set as the user's preference.

The user's preferred hand and the type of the performed procedure can, in turn, be entered manually or inferred automatically via pre-trained classifiers, by processing visual data acquired at the beginning of the surgery. The received user preference data may be received before one or more of the identifications/selections at blocks 310, 316, and 320 are made. The user preference data received at block 308 may be received from user preference data source 212.

Receiving user preference data at block 308 may include accessing a user profile (e.g., stored in user preference data source 212) electronically storing one or more user preferences associated with a user. System 200 may identify a user preference from the one or more user preferences associated with the user, wherein the identified user preference indicates a preferred functionality, and/or a preferred modality. The preferred surgical functionality and/or the preferred modality may then be selected for the user. For example, a preferred modality for a user may be chosen based on whether the user is right-handed or left-handed, for example such that the orientation of the mapping of input buttons on surgical input device 210 can be set in accordance with the user's dominant hand.

As shown at block 312, system 200 may optionally receive a confirmatory user input and use the confirmatory user input as part of the basis for confirming identification of a surgical tool at block 310, selection of a functionality for the identified surgical tool at block 316, and/or selection of a modality for surgical input device 210 at block 320. System 200 may use the confirmatory user input as part of the basis for pairing the identified surgical tool with surgical input device 210 at block 314, setting the identified surgical tool to the selected functionality at block 318, and/or setting surgical input device 210 to the selected modality at block 322.

The received confirmatory user input may comprise an instruction to confirm identification of a surgical tool, to confirm selection of a functionality, and/or to confirm selection of a modality. System 200 may cause one or more suggested identifications/selections to be displayed (e.g., by display device 216) and a user may then execute the confirmatory user input to confirm (or reject) the suggested identification(s)/selection(s). The received confirmatory user input may thus be received after one or more identifications/selections are made at blocks 310, 316, and 320 are made, but before the action(s) at one or more of blocks 314, 318, and/or 322 are taken. The confirmatory user input received at block 312 may be received via user input device 214, via surgical input device 210, and/or via any other input device. Optionally, the received confirmatory user input may comprise a manual selection by the user that may override a selection of functionality and/or force a change in functionality. For example, the user input may command a switch in pairing from one surgical tool to another surgical tool, which may override one or more of the pairings and/or settings of steps 314, 318, and/or 322.

One or more aspects of a configuration of a surgical tool and/or the surgical input device 210 according to method 300 may be displayed to the user in a graphical user interface displayed on a suitable display device, such as display device 216. For example, a graphical indication may be included in a graphical user interface indicating the surgical tool with which the surgical input device 210 was paired at step 314. For example, and with reference to FIGS. 5A and 5B, a graphical user interface 520 that include the frame 502 may include an indicator 522 that indicates that the surgical input device 210 is paired with surgical tool 500 (e.g., a drill). By displaying indicator 522 alongside the frame 502, the user can easily see which surgical tool the surgical input device 210 is paired with without having to look away from the display displaying the endoscopic video. The graphical user interface 520 may include any other information relevant to the surgical tool and/or surgical input device 210, including any functionality of the surgical tool set in step 318 and/or any modality of the surgical input device 210 set in step 322. Relevant information that may be displayed in the graphical user interface 520 includes, for example, one or more error indicators 524 indicating errors associated with the configuration of a surgical tool and/or the surgical input device 210 according to method 300.

In one example of application of the techniques described herein, a foot switch is provided for use as a surgical input device. The foot switch allows control of both a shaver hand piece (used with cutters and burs) and an RF probe. The foot switch currently has a toggle button that a user may manually press to toggle between the shaver and RF probe functionalities. In accordance with the techniques described herein, the foot switch may be configured for automatic configuration. A tool detection model may be trained using tagged images to detect RF probes, cutters, and burs, along with other tools. The model may be deployed on a connected OR hub or other edge computing device that can be connected to an image output (e.g., a video output) of an imaging device (e.g., an endoscopic imaging device). Live images (e.g., video images) may be passed through the deployed model and classification output data may be returned, wherein the inference values may provide probabilities of the image containing the different tools. The inference values may be passed to a surgical pump over SFB communication. The surgical pump may also request from the foot switch a current toggle state, or toggle position, of the foot switch using SFB communication. An algorithm on the surgical pump may compare the inference values and the current toggle position and determine if the foot switch should remain in its current toggle position or if the toggle position should be changed based on a tool detected in the one or more images (e.g., in the video). If it is determined that the toggle position of the foot switch should be changed, a command may be sent from the surgical pump to the foot switch to change the toggle position of the foot switch.

In one example application, a foot switch may be in a toggle position that allows for a shaver to be activated using the foot switch. A cutter may be replaced from an endoscopic view with an RF probe. Classification output data (e.g., including an inference value) returned by one or more classifiers analyzing the one or more images may indicate that an RF probe has been inserted into the joint space. An algorithm may determine that the toggle button should be changed for RF probe activation. A command may be sent to the foot switch to configure the foot switch for use with the RF probe. A user may now directly activate the RF probe by pressing the activation foot pedal of the foot switch without being required to manually press the toggle button on the foot switch to configure the foot switch for use with the RF probe.

In some aspects, a system leveraging the techniques disclosed herein may be configured to change a pairing, functionality, and/or modality based on one or more detected joint conditions.

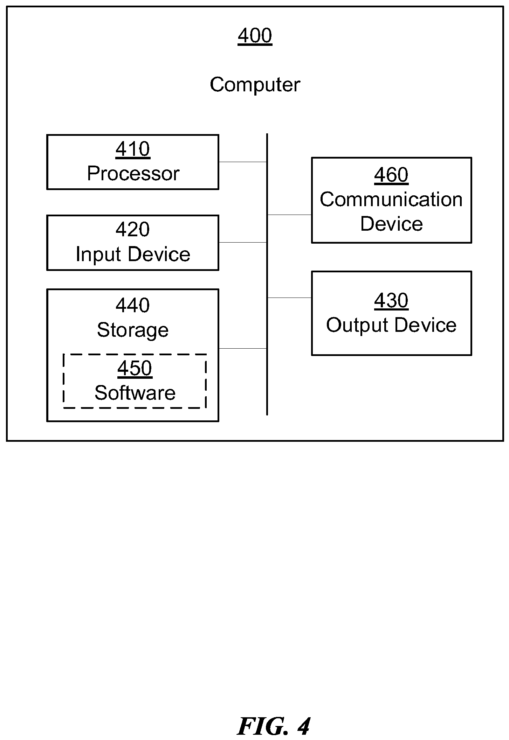

FIG. 4 illustrates an example of a computing system 400, in accordance with some examples of the disclosure. Computing system 400 can be a client or a server. As shown in FIG. 4, computing system 400 can be any suitable type of processor-based system, such as a personal computer, workstation, server, handheld computing device (portable electronic device) such as a phone or tablet, or dedicated device. The computing system 400 can include, for example, one or more of input device 420, output device 430, one or more processors 410, storage 440, and communication device 460. Input device 420 and output device 430 can either be connectable or integrated with computing system 400.

Input device 420 can be any suitable device that provides input, such as a touch screen, keyboard or keypad, mouse, gesture recognition component of a virtual/augmented reality system, or voice-recognition device. Output device 430 can be or include any suitable device that provides output, such as a display, touch screen, haptics device, virtual/augmented reality display, or speaker.

Storage 440 can be any suitable device that provides storage, such as an electrical, magnetic, or optical memory including a RAM, cache, hard drive, removable storage disk, or other non-transitory computer readable medium. Communication device 460 can include any suitable device capable of transmitting and receiving signals over a network, such as a network interface chip or device. The components of the computing system 400 can be connected in any suitable manner, such as via a physical bus or wirelessly.

Processor(s) 410 can be any suitable processor or combination of processors, including any of, or any combination of, a central processing unit (CPU), field programmable gate array (FPGA), and application-specific integrated circuit (ASIC). Software 450, which can be stored in storage 440 and executed by one or more processors 410, can include, for example, the programming that provides the functionality or portions of the functionality of the present disclosure (e.g., as described with respect to in the systems and methods as described above).

Software 450 can also be stored and/or transported within any non-transitory computer-readable storage medium for use by or in connection with an instruction execution system, apparatus, or device, such as those described above, that can fetch instructions associated with the software from the instruction execution system, apparatus, or device and execute the instructions. In the context of this disclosure, a computer-readable storage medium can be any medium, such as storage 440, that can contain or store programming for use by or in connection with an instruction execution system, apparatus, or device.

Software 450 can also be propagated within any transport medium for use by or in connection with an instruction execution system, apparatus, or device, such as those described above, that can fetch instructions associated with the software from the instruction execution system, apparatus, or device and execute the instructions. In the context of this disclosure, a transport medium can be any medium that can communicate, propagate or transport programming for use by or in connection with an instruction execution system, apparatus, or device. The transport computer readable medium can include, but is not limited to, an electronic, magnetic, optical, electromagnetic, or infrared wired or wireless propagation medium.

Computing system 400 may be connected to a network, which can be any suitable type of interconnected communication system. The network can implement any suitable communications protocol and can be secured by any suitable security protocol. The network can comprise network links of any suitable arrangement that can implement the transmission and reception of network signals, such as wireless network connections, T1 or T3 lines, cable networks, DSL, or telephone lines.

Computing system 400 can implement any operating system suitable for operating on the network. Software 450 can be written in any suitable programming language, such as C, C++, Java, or Python. In various aspects, application software providing the functionality of the present disclosure can be deployed in different configurations, such as in a client/server arrangement or through a Web browser as a Web-based application or Web service, for example.

The foregoing description, for the purpose of explanation, has been described with reference to specific aspects and examples. However, the illustrative discussions above are not intended to be exhaustive or to limit the invention to the precise forms disclosed. Many modifications and variations are possible in view of the above teachings. The aspects and examples were chosen and described in order to best explain the principles of the techniques and their practical applications. Others skilled in the art are thereby enabled to best utilize the techniques and various aspects with various modifications as are suited to the particular use contemplated.

Although the disclosure and examples have been fully described with reference to the accompanying figures, it is to be noted that various changes and modifications will become apparent to those skilled in the art. Such changes and modifications are to be understood as being included within the scope of the disclosure and examples as defined by the claims. Finally, the entire disclosure of the patents and publications referred to in this application are hereby incorporated herein by reference.

For the purpose of clarity and a concise description, features are described herein as part of the same or separate examples; however, it will be appreciated that the scope of the disclosure includes examples having combinations of all or some of the features described.

## Claims

1. A system for automatically configuring a surgical tool and a surgical input device for the surgical tool, the system comprising one or more processors configured to cause the system to:
receive one or more images;
identify, based at least in part on the one or more images, the surgical tool from a plurality of surgical tools; and
generate an instruction to pair the surgical input device with the surgical tool.

2. The system of claim 1, wherein the one or more processors are further configured to cause the system to transmit the instruction to pair the surgical input device with the surgical tool to one or both of the surgical input device and the surgical tool.

3. The system of claim 1 or claim 2, wherein the one or more processors are further configured to cause the system to, after identifying the surgical tool:
receive a confirmatory user input confirming the identification of the surgical tool; and
in response to receiving the confirmatory user input confirming the identification of the surgical tool, transmit the instruction to pair the surgical input device with the surgical tool to one or both of the surgical input device and the surgical tool;
wherein optionally the confirmatory user input confirming the identification of the surgical tool is received from the surgical input device.

4. The system of any of the preceding claims, wherein the one or more processors are configured to apply one or more classifiers to the one or more images, each of the one or more classifiers generating classification output data; and
wherein identifying the surgical tool from the plurality of surgical tools based at least in part on the one or more images comprises identifying the surgical tool from the plurality of surgical tools based at least in part on the classification output data;
wherein optionally the one or more classifiers comprise at least one of: a tool identification classifier; a tool location classifier; an anatomical identification classifier; an anatomical position classifier; a surgery type classifier; a surgery stage classifier, a tool identification classifier, and/or a tool location classifier.

5. The system of any of the proceeding claims, wherein:
the one or more processors are configured to apply one or more computer vision algorithms to the one or more images, each of the one or more computer vision algorithms generating computer vision output data; and
identifying the surgical tool from the plurality of surgical tools based at least in part on the one or more images comprises identifying the surgical tool from the plurality of surgical tools based at least in part on the computer vision output data.

6. The system of any of the proceeding claims, wherein the one or more processors are further configured to cause the system to:
select, based at least in part on the one or more images, a functionality for the surgical tool; and
generate an instruction to configure the surgical tool in accordance with the functionality;
wherein optionally a) the one or more processors are further configured to cause the system to transmit the instruction to configure the surgical tool in accordance with the functionality to the surgical tool; and/or
wherein optionally b)the one or more processors are further configured to cause the system to, after selecting the functionality for the surgical tool:
receive a confirmatory user input confirming the selection of the functionality for the surgical tool; and
in response to receiving the confirmatory user input confirming the selection of the functionality for the surgical tool, transmit the instruction to configure the surgical tool in accordance with the selected functionality to the surgical tool;
wherein further optionally the confirmatory user input is received from the surgical input device.

7. The system of claim 6, wherein:
the one or more processors are configured to cause the system to, before selecting the functionality for the surgical tool, receive data representing a user preference; and
selecting the functionality for the surgical tool is based at least in part on the data representing the user preference;
wherein optionally:
receiving the data representing the user preference comprises:
accessing a user profile electronically storing one or more user preferences associated with a user; and
identifying the user preference, from the one or more user preferences associated with the user, indicating a preferred functionality; and
selecting the functionality for the surgical tool is based at least in part on the preferred functionality.

8. The system of any of the preceding claims, wherein the one or more processors are further configured to cause the system to:
select, based at least in part on the one or more images, a modality for the surgical input device; and
generate an instruction to configure the surgical input device in accordance with the modality;
wherein optionally a) the modality is selected based at least in part on a selected functionality for the surgical tool; and/or
wherein optionally b) the one or more processors are further configured to cause the system to transmit the instruction to configure the surgical input device in accordance with the modality to the surgical input device.

9. The system of claim 8, wherein the one or more processors are further configured to cause the system to, after selecting the modality for the surgical input device:
receive a confirmatory user input confirming the selection of the modality for the surgical input device; and
in response to receiving the confirmatory user input confirming the selection of the modality for the surgical input device, transmit the instruction to configure the surgical input device in accordance with the modality to the surgical input device;
wherein optionally the confirmatory user input confirming the selection of the modality for the surgical input device is received from the surgical input device.

10. The system of claim 8 or claim 9, wherein:
the one or more processors are configured to cause the system to, before selecting the modality for the surgical input device, receive data representing a user preference; and
selecting the modality for the surgical input device is based at least in part on the data representing the user preference;
wherein optionally:
receiving the data representing the user preference comprises:
accessing a user profile electronically storing one or more user preferences associated with a user; and
identifying the user preference, from the one or more user preferences associated with the user, indicating a preferred modality; and
selecting the modality for the surgical input device is based at least in part on the preferred modality.

11. The system of any of the preceding claims, wherein the surgical input device comprises a foot switch.

12. The system of any of the preceding claims, wherein the surgical input device comprises a hand control device.

13. The system of any of the preceding claims, wherein the one or more images comprise intraoperative images captured by a surgical imaging device, the one or more images including the surgical tool within a field of view of the surgical imaging device.

14. A method for automatically configuring a surgical tool and a surgical input device for the surgical tool, the method comprising:
receiving one or more images;
identifying, based at least in part on the one or more images, the surgical tool from a plurality of surgical tools; and
generating an instruction to pair the surgical input device with the surgical tool.

15. A computer program product comprising software code portions for automatically configuring a surgical tool and a surgical input device for the surgical tool, the software code portions configured to be executed by one or more processors of a system to cause the system to:
receive one or more images;
identify, based at least in part on the one or more images, the surgical tool from a plurality of surgical tools; and
generate an instruction to pair the surgical input device with the surgical tool.
